# EUROPÄISCHE PATENTSCHRIFT

(11) **EP 0 567 464 B1**
(45) Veröffentlichungstag und Bekanntmachung des Hinweises auf die Patenterteilung: **14.08.1996**
(21) Anmeldenummer: 92900789.6
(22) Anmeldetag: 18.12.1991
(51) Int. Cl.: G01N 27/12

(54) **SENSOR FÜR DIE BESTIMMUNG VON KOHLENMONOXID**
SENSOR FOR DETERMINING CARBON MONOXIDE
CAPTEUR DE DETERMINATION DU MONOXYDE DE CARBONE

(30) Priorität: 15.01.1991 DE 4100915
(43) Veröffentlichungstag der Anmeldung: 03.11.1993
(73) Patentinhaber: ROBERT BOSCH GMBH, 70442 Stuttgart (DE)
(72) Erfinder: HUTH, Gerhard, D-7016 Gerlingen (DE); BARESEL, Detlef, D-7000 Stuttgart 70 (DE)
(86) Internationale Anmeldenummer: DE9100986
(87) Internationale Veröffentlichungsnummer: WO9213270

(56) Entgegenhaltungen:
- EP-A- 0 102 067
- EP-A- 0 141 033
- PATENT ABSTRACTS OF JAPAN vol. 10, no. 30 (P-426)(2087) 5. Februar 1986 6 JP-A-60 179 649
- PATENT ABSTRACTS OF JAPAN vol. 8, no. 214 (P-304)(1651) 29. September 1984 6 JP-A-59 097 047

## Beschreibung

Die Erfindung betrifft einen Sensor für die Bestimmung von Kohlenmonoxid, insbesondere in Sauerstoff enthaltenden Gasgemischen, auf der Basis von dotierten Metalloxiden, deren elektrische Leitfähigkeit bzw. deren elektrischer Widerstand eine Funktion der Zusammensetzung der Gasgemische ist.

### Stand der Technik

Sensoren für Kohlenmonoxid werden z.B. für die Ueberwachung von Heizanlagen auf Basis fossiler Brennstoffe oder von Verbrennungsmotoren benötigt. In beiden Fällen wird aus ökonomischen und ökologischen Gründen angestrebt, die fossilen Brennstoffe bzw. den Kraftstoff möglichst vollständig zu verbrennen. Man wendet daher die Verbrennungsluft in gewissem Ueberschuß an, so daß das Abgas noch Sauerstoff, aber trotzdem auch noch geringe Mengen an Kohlenmonoxid aufweist. Zur Optimierung der Verbrennungsvorgänge ist es nützlich, den Gehalt an Kohlenmonoxid im Abgas zu kennen.

Sensoren zur Bestimmung des Gehaltes an kohlenmonoxid in Abgasen auf der Basis von Metalloxiden, die bei den vorherrschenden hohen Temperaturen in Abhängigkeit vom Gehalt an Kohlenmonoxid, aber auch vom Sauerstoffgehalt, ihre elektrische Leitfähigkeit andern, sind bekannt. So werden in der DE-PS 26 03 785 Sensoren aus Chrom(III)-oxid oder Zinn(IV)-oxid beschrieben, die mit mindestens einem Oxid der Uebergangsmetalle der 4. bis 6. Gruppe des Periodensystems oder einem Oxid des Eisens, Nickels, Kobalts, Zinns, Magnesiums, Calciums oder Lithiums dotiert ist.

Aus der DE-PS 26 48 373 sind Sensoren mit Halbleitern bekannt, die aus dotiertem Zinn(IV)-oxid bestehen, wobei die katalytische Aktivität des Halbleiters für die Reaktion zwischen Sauerstoff und oxidierbaren Anteilen, wie Kohlenmonocid, gezielt vermindert wird. Sensoren auf Basis von Cer(IV)-oxid, das mit Magnesiumoxid, Aluminiumoxid, Yttriumoxid, Titan(IV)-oxid, Tantal(V)-oxid, Niobium(V)-oxid oder Vanadium(V)-oxid dotiert ist, werden in der DE-PS 30 24 449 beschrieben.

Schließlich findet sich in "Sensors and Actuators", 6 (1984), 35 - 50 eine Arbeit über den Einfluß der katalytischen Aktivität von halbleitenden Metalloxiden auf deren Eigenschaften als Sensoren für Kohlenmonoxid, verbunden mit einer umfassenden Uebersicht über die einschlägige Literatur.

Sensoren der beschriebenen Art haben in die Praxis Eingang gefunden und sich im großen und ganzen bewährt. Sie lassen jedoch insbesondere dann Wünsche offen, wenn es darum geht, kleine Mengen an Kohlenmonoxid in Gasgemischen zu bestimmen, deren Temperaturen schwanken. Die Sensoren müssen dann hohe Empfindlichkeit mit einem niedrigen Widerstands-Temperatur-Koeffizienten verbinden. Mit anderen Worten, einerseits sollen schon kleine Schwankungen im Kohlenmonoxidgehalt zu deutlichen Veränderungen in der Leitfähigkeit bzw. im Widerstand führen, und andererseits soll die Leitfähigkeit bzw. der Widerstand möglichst weitgehend unabhängig von der Meßtemperatur sein. Weiterhin sind die Sensoren des Standes der Technik hinsichtlich ihrer Standzeit nicht voll befriedigend. Es ist daher erwünscht, Sensoren mit längerer Lebensdauer zu entwickeln.

### Vorteile der Erfindung

Die Sensoren nach der Erfindung, wie sie in den Patentansprüchen beschrieben ist, weisen insbesondere bei geringen Kohlenmonoxidgehalten eine hohe Empfindlichkeit auf. Sie verbinden diese Eigenschaft mit einer nur geringen Abhängigkeit der Leitfähigkeit vom Gehalt an Sauerstoff. Die gemessene Leitfähigkeit wird daher weitgehend vom Gehalt an Kohlenmonoxid bestimmt . Es besteht über weite Konzentrations- bzw. Partialdruckbereiche ein annähernd linearer Zusammenhang zwischen diesen beiden Größen. Die Sensoren nach der Erfindung zeigen bei einer gegebenen Zusammensetzung des Gasgemisches im technisch bedeutsamen Temperaturbereich, etwa von 300 bis 700 °C, eine geringere Temperaturabhängigkeit der Leitfähigkeit als die bekannten Sensoren. Das gilt insbesondere, wenn sie zusätzlich mit Tantal(V)-oxid dotiert sind. Sie führen daher auch ohne aufwendige Kompensationsschaltungen, die bei Sensoren nach dem Stand der Technik bei starken Temperaturschwankungen in den Abgasen erforderlich sind, zu guten und gut reproduzierbaren meßergebnissen. Ein weiterer Vorteil der Sensoren nach der Erfindung liegt in deren verlängerter Lebensdauer. Standzeiten von 5000 und mehr Betriebsstunden können erreicht werden.

### Beschreibung der Erfindung

Die Sensoren nach der Erfindung eignen sich besonders zur Bestimmung von Kohlenmonoxid in armen, d.h. wenig Kohlenmonoxid und vergleichsweise viel Sauerstoff enthaltenden Abgasen. Die Gehalte an Kohlenmonoxid können z.B. 5 bis 1500 ppm, vorzugsweise 10 bis 1200 ppm betragen, die an Sauerstoff 2 bis 20 Vol%, vorzugsweise 2 bis 10 Voll Die überwiegenden Anteile sind Stickstoff und Kohlendioxid, doch können im Gemisch auch kleinere Mengen an Stickoxiden und/oder unverbrannten Kohlenwasserstoffen enthalten sein.

Die Bestandteile der Sensoren sind im Zusammenhang mit dotierten Halbleitern für sich alle beschrieben. Die überraschenden Eigenschaften der Sensoren nach der Erfindung sind auf die qualitative und die quantitative Auswahl zurückzuführen. Metalloxide, die eine n-Leitfähigkeit aufweisen, sind bekanntlich solche, die gegenüber der Stöchiometrie ein geringes Sauerstoffdefizit aufweisen. Beispiele hierfür sind Zinkoxid und Cer(IV)-oxid. Als gut geeignet hat sich Zinn(IV)-oxid erwiesen.

Von den Oxiden von anderen Metallen mit einer maximalen Wertigkeit < 4 seien beispielsweise Aluminiumoxid, Kupfer(II)-oxid, Eisen(II)-oxid, Eisen(III)-oxid, Nickeloxid, Kobaltoxid, Calciumoxid und Strontiumoxid erwähnt. Bevorzugt wird Magnesiumoxid verwendet. Die Metalloxide werden nur in geringen Mengen zugesetzt, insbesondere in Mengen von 0,05 bis 0,15 Mol%.

Für die Funktion der Sensoren ist es wichtig, daß die Oxidation des Kohlenmonoxids zu Kohlendioxid zwar katalysiert wird, aber nur mit geringer Geschwindigkeit, und jedenfalls langsamer abläuft als die Adsorption des Kohlenmonoxids an der mit Sauerstoff beladenen Oberfläche des n-leitenden Metalloxids. Dementsprechend wählt man den Katalysator nach Art und Menge aus. Als Oxide von Xetallen, die Reaktion von Kohlenmonoxid mit Sauerstoff zu Kohlendioxid katalysieren, eignen sich für Sensoren nach der Erfindung u.a. diejenigen der Platinmetalle Platin, Rhodium und Ruthenium sowie insbesondere Palladium ; weiterhin sind die Oxide von Mangan, Chrom, Kobalt und Nickel verwendbar. Auch diese Bestandteile werden nur in geringen Mengen zugesetzt, insbesondere in Mengen von 0,05 bis 0,15 Mol%. Für beide Dotierungsstoffe gilt, daß ihr optimaler Gehalt - innerhalb der im Anspruch 1 genannten weiteren sowie der oben aufgeführten engeren Grenzen - von der Auswahl der betreffenden Stoffe abhängt und durch Vorversuche unschwer ermittelt werden kann.

Einen besonders kleinen Widerstands-Temperatur-Koeffizienten zeigen solche Sensoren, bei denen das n-leitende Metalloxid zusätzlich ein Oxid eines Metalls der 5. Nebengruppe des Periodensystems der Elemente, also des Vanadiums, Niobiums und insbesondere des Tantals, enthält. Auch dieser Zusatzstoff wird in nur geringen Mengen, vorteilhaft 0,003 bis 0,03 Mol %, verwendet.

Wenn die beiden Dotierungsstoffe nach Anspruch 1 sowie der Zusatzstoff nach Anspruch 2 als Oxide bezeichnet werden, dann soll dies lediglich den Oxidationsgrad des Metalls bei der Herstellung des Sensors bezeichnen. Möglicherweise liegen die betreffenden Metalle in den fertigen Sensoren zumindest teilweise in Form anderer chemischer Verbindungen, z.B. als Stannate, oder aber in metallischer Form, z.B. als Palladium, vor.
Die dotierten n-leitenden Metalloxide werden in üblicher Weise hergestellt. So kann man die entsprechenden metallsalze in entsprechenden Mengen in Wasser lösen und aus der Lösung das Gemisch der Oxide, Hydroxide, Oxidhydrate und/oder Carbonate mittels alkalischer Stoffe, wie Natronlauge, Sodalösung oder wässerigem Ammoniak, ausfällen. Man kann dann das Gemisch eindicken, zur Trockene eindampfen und die wasserlöslichen Stoffe aus der festen Phase herauslösen. Die wasserlöslichen Anteile können aber auch vor dem Eindampfen durch Waschen und Dekantieren abgetrennt werden.

Das trockene dotierte n-leitende Metalloxid wird dann, gegebenenfalls nach Tempern bei 500 bis 650 °C, zweckmäßig feinteilig gemahlen, angeteigt und auf einen mit Elektroden, beispielsweise aus Platin, versehenen Träger aufgebracht. Man läßt den beschichteten Träger trocknen und aktiviert das dotierte n-leitende Metalloxid, indem man es auf dem Träger allmählich auf eine Temperatur von etwa 800 bis 1000 °C erhitzt und ebenso allmählich wieder abkühlen läßt. Der so erhaltene Sensor kann als Viderstand direkt in den Meßkreis geschaltet werden.

### Beispiel

52 g Zinn(IV)-chlorid, 40,6 mg Magnesiumchlorid-Hexahydrat, 10,6 mg Palladiumchlorid und 7,6 mg Tantal(V)-chlorid werden in 2 1 Wasser gelöst, und die Lösung wird auf 80 °C erhitzt. Unter lebhaftem Rühren wird soviel wässerige 10 %ige Ammoniaklösung mit einer Geschwindigkeit von 1 Tropfen pro Sekunde zugegeben, bis ein pH Wert von 8 erreicht ist. Die Temperatur wird auf 90 °C gesteigert und der Ansatz bei dieser Temperatur 3 Stunden gehalten, wobei das Gemisch dickflüssig wird. Man dampft den ganzen Ansatz an der Luft bei einer Badtemperatur von 13o 130 °C innerhalb von 10 Stunden zur Trockene ein. Nach dem Erkalten wird die feste Masse mit 3 l Vasser aufgeschlämmt, 4 Stunden digeriert und das Wasser dekantiert. Diese Behandlung wird mit 4 1 Wasser wiederholt. Die grobkristalline Masse wird in einem Rohrofen bei einer Temperatursteigerung von 50 °C/h auf 600 °C erhitzt und 5 Stunden auf dieser Temperatur gehalten. Man läßt die Masse mit einer Abkühlrate von 100 °C/h bis auf Raumtemperatur abkühlen, vermischt sie mit Toluol ( 15 g je 10 g trockene Masse ) und mahlt das Gemisch 24 Stunden in einer schnellaufenden Hartmetallkugelmühle. Das Mahlgut wird 4 Stunden an der Luft getrocknet, das restliche Toluol im Vakuumtrockenscharank bei 100 °C und etwa 0,01 Torr entfernt.

Zur Herstellung der auf den Aluminiumoxid-Träger aufzurakelnden Paste wird das trockene Pulver mit einem "Dicköl" ( 50 % Benzylalkohol, 30 % Ethylcellulose, 20 % Terpineol ) gemischt ; das Volumenverhältnis Pulver zu Paste beträgt etwa 1 : 1. Zur Herstellung des Sensors wird die Paste auf einen auf der Oberseite mit zwei Elektroden aus Platin und auf der Unterseite mit einem aufgedruckten Platin-Heizmäander versehenen keramischen Träger so aufgestrichen, daß die Schicht, etwa 0,1 mm stark, gleichmäßig erscheint. Man läßt den beschichteten Träger etwa 10 Minuten bei 70 °C an der Luft liegen und erhitzt ihn dann allmählich ( Temperatursteigerung etwa 20 °C /h ) auf 900 °C. Nachdem diese Temperatur 2 Stunden eingehalten wurde, läßt man den Sensor bei einer Temperaturabsenkung von etwa 100 °C/h auf Raumtemperatur abkühlen.

Aus der Zeichnung erkennt man die vorteilhaften Eigenschaften der Sensoren nach der Erfindung. Die Abb. 1 bis 3 geben die Widerstands-Temperatur-Charakteristik verschiedener Sensoren in logarithmischer Auftragung wieder. Dabei betrifft Abb. 1 einen Sensor nach dem Stand der Technik, Abb. 2 gibt die Eigenschaften eines Sensors nach Anspruch 1, Abb. 3 diejenigen eines Sensors nach Anspruch 2 wieder. Die dotierten n-leitenden Metalloxide haben die folgenden Zusammensetzungen :

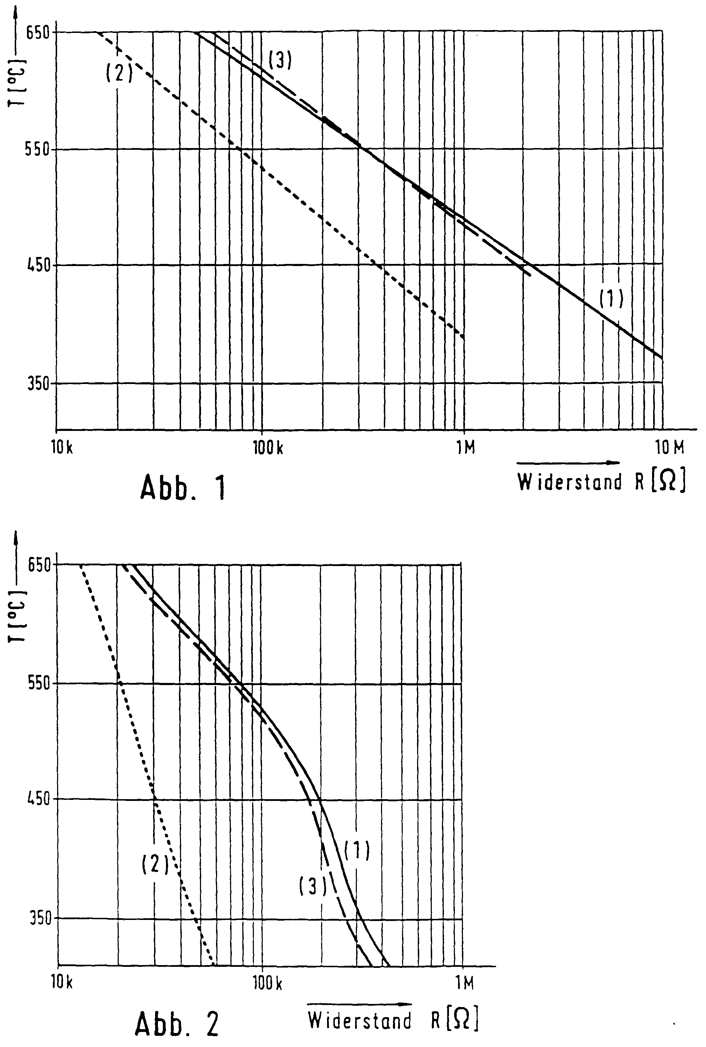

- Abb. 1 :: Zinn(IV)-oxid mit 4 Mol% Magnesiumoxid, 2 Mol% Aluminiumoxid, 3 Mol% Kupfer(II)-oxid
- Abb. 2 :: Zinn(IV)-oxid mit 0,10 Mol% Magnesiumoxid, 0,14 Mol% Palladiumoxid

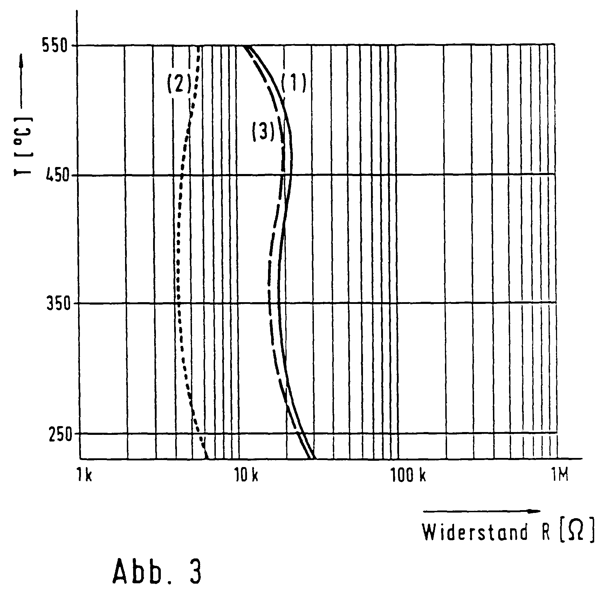

- Abb. 3 :: Zinn(IV)-oxid mit 0,10 Mol% Magnesiumoxid, 0,10 Mol% Palladiumoxid, 0,005 Mol% Tantal(V)-oxid

Alle Abbildungen zeigen 3 Kurven, die in der Reihenfolge der Ziffern gemessen wurden, und zwar die erste Kurve mit einem Gasgemisch aus
5 Vol% Sauerstoff, Rest Stickstoff,
und die zweite Kurve mit einem Gasgemisch aus
5 Vol% Sauerstoff, 1000 ppm Kohlenmonoxid, Rest Stickstoff.

Die dritte Kurve gibt Messungen mit dem Gasgemisch der ersten Kurve wieder.

Man erkennt, daß die Sensoren nach der Erfindung keine nennenswerte Hysterese, wohl aber eine hohe Empfindlichkeit für Kohlenmonoxid sowie insbesondere eine gemessen am Stand der Technik deutlich geringere Abhängigkeit des Widerstandes bzw. der Leitfähigkeit von der Temperatur zeigen. Dies gilt besonders für den Sensor nach Abb. 3.

## Patentansprüche

1. Sensor für die Bestimmung von Kohlenmonoxid, insbesondere in Sauerstoff enthaltenden Gasgemischen auf der Basis von dotierten n-leitenden Metalloxiden, deren elektrische Leitfähigkeit eine Funktion der Zusammensetzung der Gasgemische ist, dadurch gekennzeichnet, daß als Dotierungsstoffe (a) 0,01 bis 0,2 Mol% eines Oxids eines anderen Metalls mit einer maximalen Wertigkeit < 4 zusammen mit (b) 0,01 bis 0,2 Mol% eines Oxids eines Metalls, das die Reaktion von Kohlenmonoxid zu Kohlendioxid katalysiert und (c) 0,001 bis 0,1 Mol% eines Oxids eines Metalls der 5. Nebengruppe des Periodensystems der Elemente eingesetzt werden.

2. Sensor nach Anspruch 1, dadurch gekennzeichnet, daß er als n-leitendes Metalloxid Zinn(IV)-oxid enthält, das mit Magnesiumoxid Palladiumoxid und dem Oxid eines Metalls der 5. Nebengruppe des Periodensystems der Elemente dotiert ist.

3. Sensor nach einem der Ansprüche 1 oder 2, dadurch gekennzeichnet, daß er Tantal(V)-oxid als Oxid eines Metalls der 5. Nebengruppe des Periodensystems der Elemente enthält.

## Claims

1. Sensor for the determination of carbon monoxide, especially in gas mixtures containing oxygen, on the basis of doped n-type metal oxides whose electric conductivity is a function of the composition of the gas mixtures, characterised in that the dopants used comprise (a) from 0.01 to 0.2 mol% of an oxide of another metal having a maximum valency < 4 together with (b) from 0.01 to 0.2 mol% of an oxide of a metal, which catalyses the reaction of carbon monoxide to carbon dioxide and (c) from 0.001 to 0.1 mol% of an oxide of a metal of the 5th subgroup of the Periodic Table of the Elements.

2. Sensor according to Claim 1, characterised in that it comprises, as the n-type metal oxide, tin(IV) oxide which is doped with magnesium oxide, palladium oxide and the oxide of a metal of the 5th subgroup of the Periodic Table of the Elements.

3. Sensor according to either of Claims 1 or 2, characterised in that it comprises tantalum(V) oxide as the oxide of a metal of the 5th subgroup of the Periodic Table of the Elements.

## Revendications

1. Capteur pour la détermination de la teneur en monoxyde de carbone, en particulier dans des mélanges de gaz contenant de l'oxygène à base d'oxydes métalliques dopés à conduction de type n, dont la conductibilité électrique est une fonction de la composition du mélange des gaz, capteur caractérisé en ce qu'on emploie comme matières de dopage (a) 0,01 à 0,2 Mol% d'un oxyde d'un autre métal avec une valence maximale <4, en même temps qu'avec (b) 0,01 à 0,2 Mol% d'un oxyde d'un métal qui catalyse la réaction de transformation du monoxyde de carbone en dioxyde de carbone et (c) 0,001 à 0,1 Mol% d'un oxyde d'un métal du cinquième groupe auxiliaire de la classification périodique des éléments.

2. Capteur selon la revendication 1, caractérisé en ce qu'il contient, comme oxyde de métal à conduction de type n, de l'oxyde d'étain (IV) dopé par de l'oxyde de magnésium, de l'oxyde de palladium et de l'oxyde d'un métal du cinquième groupe auxiliaire de la classification périodique des éléments.

3. Capteur selon l'une des revendications 1 à 2, caractérisé en ce qu'il contient de l'oxyde de tantale (V) comme oxyde d'un métal du cinquième groupe auxiliaire de la classification périodique des éléments.
